# EUROPEAN PATENT APPLICATION

(11) **EP 4 134 670 A1**
(43) Date of publication of application: **15.02.2023**
(21) Application number: 21190254.9
(22) Date of filing: 09.08.2021
(51) Int. Cl.: G01N 33/49

(54) **A HEMATOLOGY ANALYZER COMPRISING A SINGLE HARDWARE MODULE AND INTEGRATED WORKFLOW**

(71) Applicant: Siemens Healthcare Diagnostics Inc., Tarrytown, NY 10591 (US)
(72) Inventor: Mach, Tivadar, 90409 Nürnberg (DE); Paulicka, Peter, 91341 Röttenbach (DE); Seliger, Ramona, 91346 Wiesenttal-Muggendorf (DE); Stelling, Frederick, Newburgh, 12550 (US)
(74) Representative: Maier, Daniel Oliver

(57) **Abstract**

The disclosure relates to a device and method for analyzing a blood sample, particularly including pre-analytical (qualification), analytical and post-analytical (verification) testing. More particularly the disclosure relates in certain embodiments to a device configured for collecting, imaging and lysing blood cells in a sample vessel by means of ultrasonic acoustic waves, generated in the vessel by an acoustic transducer driven at one particular excitation frequency or more particular excitation frequencies, or one range of frequency or ranges of frequencies. In some non-limiting embodiments, the ultrasonic acoustic waves are generated by a single acoustic transducer. Further on, according to certain embodiments, the device and workflow are configured for three analytical steps in single device, comprising pre-analytical (qualification), analytical and post-analytical (verification) testing.

## Description

### FIELD OF THE DISCLOSURE

The disclosure relates to a device and method for analyzing a blood sample, particularly including pre-analytical (qualification), analytical and post-analytical (verification) testing. More particularly the disclosure relates in certain embodiments to a device configured for collecting, imaging and lysing blood cells in a sample vessel by means of ultrasonic acoustic waves, generated in the vessel by an acoustic transducer driven at one particular excitation frequency or more particular excitation frequencies, or one range of frequency or ranges of frequencies. In some non-limiting embodiments, the ultrasonic acoustic waves are generated by a single acoustic transducer. Further on, according to certain embodiments, the device and workflow are configured for three analytical steps in single device, comprising pre-analytical (qualification), analytical and post-analytical (verification) testing.

### BACKGROUND

A significant portion of blood samples forwarded for hematology analysis, especially in central lab settings, are affected by interference arising from hemolysis, lipemia and coagulation in the samples. Most of these interferences arise from incorrect withdrawal, storage, transport or handling of the samples, and not from pathologies. However, even when samples are pathological, these indicators need to be flagged for a correct interpretation of further testing results. Usually, especially affected in samples are hematocrit (HCT), packed cell volume (PCV), RBC (red blood cell) count, hemoglobin-related indices and platelet count.

In order to avoid problems from interferences and pathologies, the primary solution is strict adherence to protocols for sample collection and handling by clinical staff. Furthermore, stand-alone analyzers exist for the relevant parameters, but for those cost and time to result are generally unattractive. Also, the evaluation of the totality of analysis results presented by a hematology analyzer by a skilled technician can highlight unusual patterns that result in the sample being re-measured, again requiring time and (human) resources. Furthermore, obvious clotting and incorrect handling are sometimes flagged by visual inspection of the blood tubes by a skilled technician, but this is not routinely carried out or not even possible in all cases.

In summary, pre-analysis can be done as a separate step, using either the blood analysis results and the experience of a lab technician, or a separate cartridge. In practice both are either prohibitively expensive, or do not preclude the sample from going through a full test run, thereby not saving test time and/or cost.

Furthermore, also the following methods are contemplated for blood analysis.

EP 3 575 848 relates to an analyzer, e.g. hematology analyzer, for analyzing a medical sample, e.g. blood sample of e.g. a human, comprising a light-field camera which is provided to capture the light field from an object region and is arranged on a microscope.

EP 3 540 631 discloses an in-vitro method for label-free determination of the cell type of white blood cells in a biological sample, such as blood, for use in the diagnosis of pathological specimens, such as leukemia, and involves determining the cell type of the white blood cell
WO 2017/157445 A1 discloses a flow cell for simultaneously sorting a first biological entity from a second biological entity using an acoustic transducer which generates a standing acoustic wave with a pressure node linearly arranged along an axis passing through a desired region.

It would be advantageous to screen samples, particularly for hemolysis (extracellular hemoglobin content), lipemia (extracellular lipid content, especially extracellular triglyceride / lipoprotein), and small clots, as a pre-test prior to running the sample on a hematological analyzer - or at least to provide a flag with these values accompanying the analysis data to make unreliable results more noticeable.

If affected samples are routed past the analysis stage, this would save the analysis time and cost, and if affected samples are flagged, this can eventually prevent false diagnoses or highlight the need to a new blood draw.

For a workflow verification, it would be potentially also advantageous to provide a control for these values, comparing e.g. the extracellular, intracellular and total concentrations of analytes to control for any discrepancies in the measurement device or the sample, and flag discrepancies for further scrutiny.

### Summary of the invention

The inventors found an integrated workflow in a single fluidic channel that achieves a time- and cost-effective pre-analytical test, analysis, and post-analytical control for a hematology analysis workflow.

A first aspect of the present invention relates to a method of analyzing a blood sample, comprising
a-i) providing a blood sample obtained from a patient;
a-ii) introducing a first part of the blood sample into a fluidic channel;
a-iii) focusing cells of the first part of the blood sample inside the fluidic channel into one region of the fluidic channel;
a-iv) analyzing the first part of the blood sample in the fluidic channel in a region different from the region where the cells are focused, particularly a cell-free region, wherein analysis includes measuring at least one property w of the blood sample, particularly at least one of extracellular hemoglobin content x, extracellular lipid content y, and maximum length of clots z;
a-v) automatically outputting at least a preliminary result of the analysis of the first part of the blood sample, wherein the preliminary result includes the at least one property w of the blood sample, particularly at least one result of an extracellular hemoglobin content x, an extracellular lipid content y, and a maximum length of clots z,
wherein if the result of the at least one property w of the blood is equal to or below a reference value, particularly if the result for extracellular hemoglobin content x is equal to or below a value of 150 mg/dL, and/or the result of extracellular lipid content y is equal to or below a value of 600 mg/dL, and/or the maximum length of clots z is equal to or less than 20 µm, the following steps are carried out: b1-i) flushing the first part of the blood sample from the fluidic channel;
b1-ii) introducing a second part of the blood sample into the fluidic channel;
b1-iii) settling cells of the second part of the blood sample inside at least a part of the fluidic channel;
b1-iv) optionally analyzing the settled cells at least for cell types;
b1-v) lysing at least red blood cells of the settled cells; and
b1-vi) analyzing at least the lysed red blood cells; or
wherein if the result of the at least one property w of the blood sample is above a reference value, particularly if the result for extracellular hemoglobin content x is above a value of 150 mg/dL, and/or the result of extracellular lipid content y is above a value of 600 mg/dL, and/or the maximum length of clots z is more than 20 µm, the following steps are carried out:
b2-i a) flushing the first part of the blood sample from the fluidic channel;
b2-ii a) automatically outputting a result that the blood sample is to be discarded and a new blood sample may need to be provided; or
b2-i b) flushing the first part of the blood sample from the fluidic channel;
b2-ii b) introducing a second part of the blood sample into the fluidic channel;
b2-iii b) settling of cells of the second part of the blood sample inside at least a part of the fluidic channel;
b2-iv b) optionally analyzing the settled cells at least for cell types;
b2-v b) lysing at least red blood cells of the settled cells;
b2-vi b) analyzing at least the lysed red blood cells, and
b2-vii b) outputting a result wherein it is indicated that the result may be erroneous.

The provided blood sample from a patient was obtained from the patient. That means that the blood sample was obtained from the patient prior to performing the method for analyzing the blood sample. Thus, the actual process of obtaining the blood sample from the patient is not part of the present method for analyzing the blood sample.

In a second aspect, the present invention relates to a device for analyzing a blood sample, comprising
- a fluidic channel;
- a first introducing element configured to introduce a first part of the blood sample into the fluidic channel;
- a focusing unit configured to focus cells of the first part of the blood sample inside the fluidic channel into one predetermined region of the fluidic channel;
- at least a first analyzing unit configured to analyze the first part of the blood sample in a region different from the region where the cells are focused, including measuring at least one property w of the blood sample, particularly at least one of an extracellular hemoglobin content x, an extracellular lipid content y, and a maximum length z of clots; and
- an outputting interface configured to automatically output at least a preliminary result of the analysis of the blood sample, wherein the preliminary result includes at least one property w of the blood sample, particularly at least one result of the extracellular hemoglobin content x, the extracellular lipid content y, and the maximum length z of clots, further comprising
- a decision module configured to compare a measured result of the at least one property w of the blood sample to a reference value, particularly at least one of the extracellular hemoglobin content x, the extracellular lipid content y, and the maximum length z of clots, to a reference value of 150 mg/dL of extracellular hemoglobin content, a reference value of 600 mg/dL of extracellular lipid content, and a reference value of a maximum length of clots of 20 µm, respectively, wherein
   if the result of the at least one property w of the blood sample is equal to or below the reference value, particularly if the result for extracellular hemoglobin content x is equal to or below the reference value of 150 mg/dL, and/or the result of extracellular lipid content y is equal to or below the reference value of 600 mg/dL, and/or the maximum length of clots z is equal to or less than 20 µm, the decision module is configured to provide an indication in the outputting interface that a second part of the blood sample can be measured, or is configured to provide no indication in the outputting interface, or
   if the result of the at least one property w of the blood sample is above the reference value, particularly if the result for extracellular hemoglobin content x is above a reference value of 150 mg/dL, and/or the result of extracellular lipid content y is above a reference value of 600 mg/dL, and/or the maximum length of clots z is above 20 µm, the decision module is configured to provide an indication in the outputting interface that the blood sample is to be discarded and a new blood sample may need to be provided, or that the result may be erroneous;
   further wherein the device comprises a second introducing element configured to introduce a second part of the blood sample into the fluidic channel, or wherein the first introducing element is further configured to also introduce a second part of the blood sample into the fluidic channel; and
   further wherein the device comprises a lysing unit configured to lyse at least red blood cells settled inside at least a part of the fluidic channel; or wherein the focusing unit is further configured to lyse at least red blood cells settled inside at least a part of the fluidic channel.

A third aspect of the present invention is directed to a non-transient computer program product, configured to be used in a device of the present invention for analyzing a blood sample, wherein the non-transient computer program product is configured to carry out the following steps:
a-ii) introducing a first part of the blood sample into a fluidic channel;
a-iii) focusing cells of the first part of the blood sample inside the fluidic channel into one region of the fluidic channel;
a-iv) analyzing the first part of the blood sample in the fluidic channel in a region different from the region where the cells are focused, particularly a cell-free region, wherein analysis includes measuring at least one property w of the blood sample, particularly at least one of extracellular hemoglobin content x, extracellular lipid content y, and maximum length of clots z;
a-v) automatically outputting at least a preliminary result of the analysis of the first part of the blood sample, wherein the preliminary result includes the at least one property w of the blood sample, particularly at least one result of an extracellular hemoglobin content x, an extracellular lipid content y, and a maximum length of clots z,
wherein if the result of the at least one property w of the blood is equal to or below a reference value, particularly if the result for extracellular hemoglobin content x is equal to or below a value of 150 mg/dL, and/or the result of extracellular lipid content y is equal to or below a value of 600 mg/dL, and/or the maximum length of clots z is equal to or less than 20 µm, the following steps are carried out: b1-i) flushing the first part of the blood sample from the fluidic channel;
b1-ii) introducing a second part of the blood sample into the fluidic channel;
b1-iii) settling cells of the second part of the blood sample inside at least a part of the fluidic channel;
b1-iv) optionally analyzing the settled cells at least for cell types;
b1-v) lysing at least red blood cells of the settled cells; and
b1-vi) analyzing at least the lysed red blood cells; or
wherein if the result of the at least one property w of the blood sample is above a reference value, particularly if the result for extracellular hemoglobin content x is above a value of 150 mg/dL, and/or the result of extracellular lipid content y is above a value of 600 mg/dL, and/or the maximum length of clots z is more than 20 µm, the following steps are carried out:
b2-i a) flushing the first part of the blood sample from the fluidic channel;
b2-ii a) automatically outputting a result that the blood sample is to be discarded and a new blood sample may need to be provided; or
b2-i b) flushing the first part of the blood sample from the fluidic channel;
b2-ii b) introducing a second part of the blood sample into the fluidic channel;
b2-iii b) settling of cells of the second part of the blood sample inside at least a part of the fluidic channel;
b2-iv b) optionally analyzing the settled cells at least for cell types;
b2-v b) lysing at least red blood cells of the settled cells;
b2-vi b) analyzing at least the lysed red blood cells, and
b2-vii b) outputting a result wherein it is indicated that the result may be erroneous.

Further aspects and embodiments of the invention are disclosed in the dependent claims and can be taken from the following description, figures and examples, without being limited thereto.

### Figures

The enclosed drawings should illustrate embodiments of the present invention and convey a further understanding thereof. In connection with the description they serve as explanation of concepts and principles of the invention. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.
Figures 1 to 4 show schematically methods of analyzing a blood sample of the present invention, respectively certain aspects thereof.
In Figures 5 and 6 devices for analyzing a blood sample according to the present invention are depicted schematically.
Figures 7 to 9 show schematically details of certain aspects of the method and device for analyzing a blood sample of the present invention.

### Detailed description of the invention

### Definitions

Unless defined otherwise, technical and scientific terms used herein have the same meaning as commonly understood by one of ordinary skill in the art to which this invention belongs.

In the context of the present invention a blood sample is a sample which is provided from a patient, and is particularly untreated. It is particularly a whole blood sample.

The patient within the scope of the present invention is not particularly restricted. According to certain embodiments, the patient in the present methods is a vertebrate, more preferably a mammal and most preferred a human patient.

A vertebrate within the present invention refers to animals having a vertebrae, which includes mammals - including humans, birds, reptiles, amphibians and fishes. The present invention thus is not only suitable for human medicine, but also for veterinary medicine.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements, but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

The use of the term "at least one" or "one or more" will be understood to include one as well as any quantity more than one. In addition, the use of the phrase "at least one of X, Y, and Z" will be understood to include X alone, Y alone, and Z alone, as well as any combination of X, Y, and Z.

The use of ordinal number terminology (i.e., "first", "second", "third", "fourth", etc.) is solely for the purpose of differentiating between two or more items and, unless explicitly stated otherwise, is not meant to imply any sequence or order or importance to one item over another or any order of addition.

A first aspect of the present invention relates to a method of analyzing a blood sample, comprising
a-i) providing a blood sample obtained from a patient;
a-ii) introducing a first part of the blood sample into a fluidic channel;
a-iii) focusing cells of the first part of the blood sample inside the fluidic channel into one region of the fluidic channel;
a-iv) analyzing the first part of the blood sample in the fluidic channel in a region different from the region where the cells are focused, particularly a cell-free region, wherein analysis includes measuring at least one property w of the blood sample, particularly at least one of extracellular hemoglobin content x, extracellular lipid content y, and maximum length of clots z;
a-v) automatically outputting at least a preliminary result of the analysis of the first part of the blood sample, wherein the preliminary result includes the at least one property w of the blood sample, particularly at least one result of an extracellular hemoglobin content x, an extracellular lipid content y, and a maximum length of clots z,
wherein if the result of the at least one property w of the blood is equal to or below a reference value, particularly if the result for extracellular hemoglobin content x is equal to or below a value of 150 mg/dL, and/or the result of extracellular lipid content y is equal to or below a value of 600 mg/dL, and/or the maximum length of clots z is equal to or less than 20 µm, the following steps are carried out:
b1-i) flushing the first part of the blood sample from the fluidic channel;
b1-ii) introducing a second part of the blood sample into the fluidic channel;
b1-iii) settling cells of the second part of the blood sample inside at least a part of the fluidic channel;
b1-iv) optionally analyzing the settled cells at least for cell types;
b1-v) lysing at least red blood cells of the settled cells; and
b1-vi) analyzing at least the lysed red blood cells; or
wherein if the result of the at least one property w of the blood sample is above a reference value, particularly if the result for extracellular hemoglobin content x is above a value of 150 mg/dL, and/or the result of extracellular lipid content y is above a value of 600 mg/dL, and/or the maximum length of clots z is more than 20 µm, the following steps are carried out:
b2-i a) flushing the first part of the blood sample from the fluidic channel;
b2-ii a) automatically outputting a result that the blood sample is to be discarded and a new blood sample may need to be provided; or
b2-i b) flushing the first part of the blood sample from the fluidic channel;
b2-ii b) introducing a second part of the blood sample into the fluidic channel;
b2-iii b) settling of cells of the second part of the blood sample inside at least a part of the fluidic channel;
b2-iv b) optionally analyzing the settled cells at least for cell types;
b2-v b) lysing at least red blood cells of the settled cells;
b2-vi b) analyzing at least the lysed red blood cells, and
b2-vii b) outputting a result wherein it is indicated that the result may be erroneous.

In the present method steps a-i) to a-v) are particularly carried out in this order. Thereafter, depending on the preliminary result of the analysis of the first part of the blood sample, wherein the preliminary result includes the at least one property w of the blood sample, particularly at least one result of an extracellular hemoglobin content x, an extracellular lipid content y, and a maximum length of clots z, one of three different variations are followed.

In a first variant, if the result of the at least one property w of the blood is equal to or below a reference value, particularly if the result for extracellular hemoglobin content x is equal to or below a value of 150 mg/dL, and/or the result of extracellular lipid content y is equal to or below a value of 600 mg/dL, and/or the maximum length of clots z is equal to or less than 20 µm, the following steps are carried out, particularly in this order: b1-i) flushing the first part of the blood sample from the fluidic channel;
b1-ii) introducing a second part of the blood sample into the fluidic channel;
b1-iii) settling cells of the second part of the blood sample inside at least a part of the fluidic channel;
b1-iv) optionally analyzing the settled cells at least for cell types;
b1-v) lysing at least red blood cells of the settled cells; and
b1-vi) analyzing at least the lysed red blood cells.

Particular good results are obtained if the result for extracellular hemoglobin content x is equal to or below a value of 150 mg/dL, particularly equal to or below a value of 120 mg/dL, and/or the result of extracellular lipid content y is equal to or below a value of 600 mg/dL, particularly equal to or below a value of 480 mg/dL, and/or the maximum length of clots z is equal to or less than 20 µm, particularly equal to or less than 15 µm.

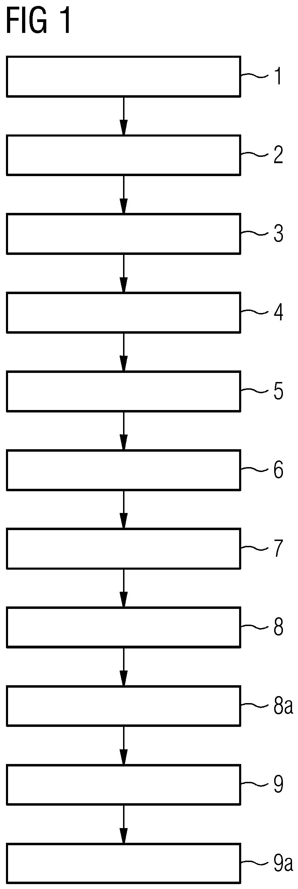

An exemplary method of the first variant is shown schematically in Fig. 1. Following step a-i) of providing a blood sample obtained from a patient 1 is step a-ii) of introducing a first part of the blood sample into a fluidic channel 2. Thereafter, step a-iii) of focusing cells of the first part of the blood sample inside the fluidic channel into one region of the fluidic channel 3 is carried out, followed by step a-iv) of analyzing the first part of the blood sample in the fluidic channel in a region different from the region where the cells are focused 4, and step a-v) of automatically outputting at least a preliminary result of the analysis of the first part of the blood sample 5.

After this follow step b1-i) of flushing the first part of the blood sample from the fluidic channel 6, step b1-ii) of introducing a second part of the blood sample into the fluidic channel 7, step b1-iii) of settling cells of the second part of the blood sample inside at least a part of the fluidic channel 8, optional step b1-iv) of analyzing the settled cells at least for cell types 8a, step b1-v) of lysing at least red blood cells of the settled cells 9, and step b1-vi) of analyzing at least the lysed red blood cells 9a.

In a second variant, if the result of the at least one property w of the blood sample is above a reference value, e.g. w1, particularly if the result is above double the reference value, e.g. 2 x w1, particularly if the result for extracellular hemoglobin content x is above a value of 150 mg/dL, further particularly if the result for extracellular hemoglobin content x is above a value of 200 mg/dL, particularly above 300 mg/dL, and/or the result of extracellular lipid content y is above a value of 600 mg/dL, further particularly if the result of extracellular lipid content y is above a value of 800 mg/dL, particularly above 1200 mg/dL, and/or the maximum length of clots z is more than 20 µm, further particularly if the maximum length of cloths is more than 25 µm, particularly above 40 µm, the following steps are carried out, particularly in this order:
b2-i a) flushing the first part of the blood sample from the fluidic channel;
b2-ii a) automatically outputting a result that the blood sample is to be discarded and a new blood sample may need to be provided.

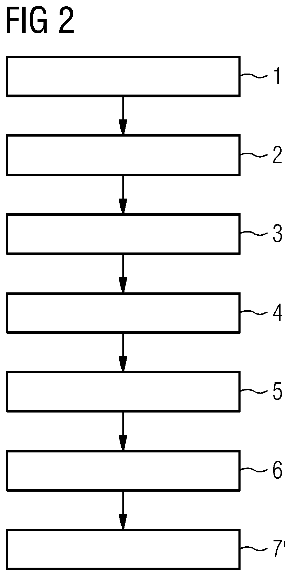

Fig. 2 shows schematically an exemplary method of the second variant, wherein steps 1 to 5 are as in Fig. 1. Thereafter step b2-i a) of flushing the first part of the blood sample from the fluidic channel 6 and step b2-ii a) of automatically outputting a result that the blood sample is to be discarded and a new blood sample may need to be provided 7' are carried out.

In a third variant, if the result of the at least one property w of the blood sample is above a reference value, e.g. w1, particularly if the result is above the reference value, e.g. w1, and equal to or below double the reference value, e.g. 2 x w1,
particularly if the result for extracellular hemoglobin content x is above a value of 150 mg/dL, further particularly if the result for extracellular hemoglobin content x is above a value of 150 mg/dL and equal to or below 300 mg/dL, particularly equal to or below 200 mg/dL, and/or the result of extracellular lipid content y is above a value of 600 mg/dL, further particularly if the result of extracellular lipid content y is above a value of 600 mg/ dL and equal to or below 1200 mg/dL, particularly equal to or below 800 mg/dL, and/or
the maximum length of clots z is more than 20 µm, further particularly if the maximum length of cloths is more than 20 µm and equal to or less than 40 µm, particularly equal to or less than 25 µm, the following steps are carried out, particularly in this order:
   b2-i b) flushing the first part of the blood sample from the fluidic channel;
   b2-ii b) introducing a second part of the blood sample into the fluidic channel;
   b2-iii b) settling of cells of the second part of the blood sample inside at least a part of the fluidic channel;
   b2-iv b) optionally analyzing the settled cells at least for cell types;
   b2-v b) lysing at least red blood cells of the settled cells;
   b2-vi b) analyzing at least the lysed red blood cells, and
   b2-vii b) outputting a result wherein it is indicated that the result may be erroneous.

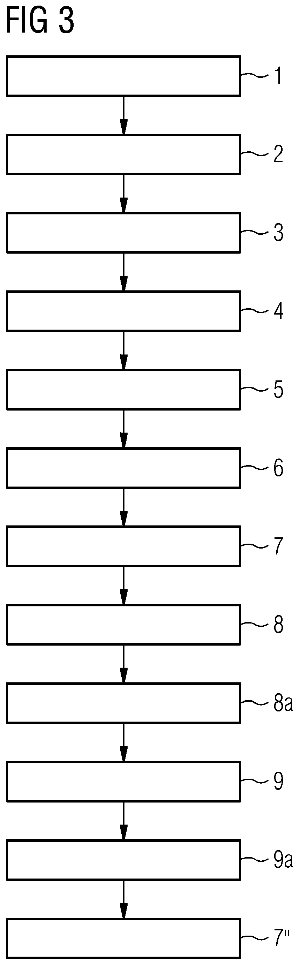

Fig. 3 schematically shows an exemplary method of the third variant, with again steps 1 to 5 being as in Fig. 1. Thereafter follows step b2-i b) of flushing the first part of the blood sample from the fluidic channel 6, step b2-ii b) of introducing a second part of the blood sample into the fluidic channel 7, step b2-iii b) of settling of cells of the second part of the blood sample inside at least a part of the fluidic channel 8, optional step b2-iv b) of analyzing the settled cells at least for cell types 8a, step b2-v b) of lysing at least red blood cells of the settled cells 9, step b2-vi b) of analyzing at least the lysed red blood cells 9a, and step b2-vii b) of outputting a result wherein it is indicated that the result may be erroneous 7".

According to preferred embodiments, the optional step of analyzing the settled cells at least for cell types is carried out, i.e. step b1-iv) or b2-iv b) is carried out.

As can be seen from the present method, steps a-i) to a-v) will always be carried out. They represent the pre-analytical part of the present method, and also contain already some analysis of the blood sample. In the pre-analytical phase of the method it is decided whether the blood sample is actually suitable for testing in the following (further) analysis steps, i.e. carrying out steps b1-i) to b1-vi); if it is maybe suitable and analysis is carried out nevertheless to have further indicators if a further blood sample is needed or not, i.e. carrying out steps b2-i b) to b2-vii b); or if it is unsuitable and a new blood sample may be needed, i.e. carrying out steps b2-i a and b2-ii a).

In the methods where the blood sample is suitable or maybe suitable, a second part of the blood sample is introduced after flushing the first part, which can be analyzed for cell types - which represents a further analysis step that is preferably carried out. At any rate the red blood cells of the second part of the blood sample are lysed and the lysed red blood cells are analyzed, which of course provides further analysis data, but also represents post-analytical steps as the results from the analysis of the lysed red blood cells can also be used for verifying the other analysis data. Thus, if the lysis is carried out, pre-analytical, analytical and post-analytical steps may be carried out on suitable or at least maybe suitable samples.

In the present method, the step a-i) of providing the blood sample from a patient is not particularly restricted. Particularly, the blood sample is provided in such a manner that no invasive action on the patient is taken, i.e. the blood sample is provided non-invasive.

Also the step a-ii) of introducing the first part of the blood sample into a fluidic channel is not particularly restricted. According to certain embodiments, the introducing of the first part of the blood sample into the fluidic channel is done automatically. According to certain embodiments, the first part of the blood sample is introduced from a syringe or a similar device, a reservoir, e.g. also on a chip, e.g. microchip that also contains the fluidic channel, etc., via a tube, separate channel, etc., using a pump, an automatic device for pressing the syringe, etc. The syringe with a tube, the reservoir with e.g. a tube and a pump, etc. therein then can form a first introducing element.

Alternatively, it is of course also possible to introduce the first and second part of the blood sample with the same first introducing element. In such case the first part can be introduced by the first introducing element in the step of introducing the first part of the blood sample into the fluidic channel, while keeping at least a second part in at least part of the first introducing element, e.g. a syringe or a reservoir. Then, a second part of the blood sample can be introduced at a later time in the step of introducing the second part of the blood sample into the fluidic channel, e.g. using the same mechanism as used for introducing the first part, e.g. pressure on the syringe, pumping from a reservoir, etc.

According to certain embodiments, the blood sample is pumped into the channel, preferably with an optical read-out. The optical read-out allows introducing only a suitable amount of blood sample. For example, if the fluidic channel is a microchannel, small volume amounts of the first and/or second blood sample may be sufficient. According to certain embodiments, less than 200 µL, less than 100 µL or even less than 70 µL, e.g. less than 50 µL, of the first part of the blood sample are introduced, and/or less than 200 µL, less than 100 µL or even less than 70 µL, e.g. less than 50 µL, of the second part of the blood sample are introduced, allowing providing of very small amounts of blood samples from the patients.

The fluidic channel is not particularly restricted, as long as it comprises a structure having inner surfaces through which the blood sample can be flown, wherein the structure is configured to contain the blood sample introduced. According to certain embodiments the fluidic channel is a microchannel, particularly in a suitable sample vessel, made from a suitable material, e.g. glass and/or a suitable polymer resin, for analysis of the blood sample, e.g. with suitable transmissivity for a spectroscopic and/or microscopic analysis for analyzing the first part of the blood sample in the fluidic channel in a region different from the region where the cells are focused, optionally analyzing the settled cells at least for cell types, and/or analyzing at least the lysed red blood cells. Alternatively, it can also be a pipe, etc., but preferably comprises at least two parallel inner and outer surfaces for focusing and/or settling cells and/or for ease of analysis. According to preferred embodiments, the fluidic channel is a microchannel in a suitable sample vessel, e.g. inside a chip made e.g. from glass, plastic, etc.

A suitable sample vessel, according to certain embodiments, has an outer surface, the fluidic channel, e.g. a microchannel, within the confines of the outer surface, a first port extending through the outer surface to the microchannel, and a second port extending through the outer surface to the microchannel, such that parts of the blood sample can be introduced through the first port into the microchannel and can be flushed from the second port. According to certain embodiments, the fluidic channel, particularly a microchannel, has a length, a width and a height, wherein preferably an aspect ratio of the width to the height is in a range from approximately 0.04 to approximately 0.175, and/or wherein the sample vessel has a width and a height, and wherein a sample vessel aspect ratio of the width to the height is in a range from approximately 0.5 to approximately 3.0. According to certain embodiments the fluidic channel is a microchannel with a height of between and including 5 and 500 µm, preferably between and including 10 and 200 µm, further preferably between and including 50 and 100 µm.

According to certain embodiments, the blood sample is separated at least into a first part and a second part prior to introducing the first part into the fluidic channel. The separation of the blood sample into at least a first part and a second part is not particularly restricted. In such cases the first part and second part of the blood sample can be introduced to the fluidic channel by a first introducing element and a second introducing element, wherein the second introducing element can be configured the same way as the first introducing element, e.g. as a syringe and tube, a reservoir with a tube and pump, etc. However, it is also possible to provide the blood sample and then introduce it only part by part, e.g. introduce the first part in step a-ii) and introduce the second part in step b1-ii) or step b2-ii b) from the same introducing element, i.e. the first introducing element and the second introducing element, e.g. a syringe, etc. According to certain embodiments also parts of the blood sample can be used for flushing the first part of the blood sample in step b1-i), b2-i a) or b2-i b), and/or for flushing the second part of the blood sample in an optional flushing step, e.g. after step b1-vi), or after step b2-vi b) or b2-vii b).

According to certain embodiments, the blood sample, or the first part and/or the second part of the blood sample, can be diluted prior to introducing. Thus, it is possible that the blood sample is diluted, and then a first part of the blood sample and a second part of the blood sample, in the first and third variant, are introduced in diluted form, i.e. using the same diluent. Alternatively, it is also possible to separate the blood sample in at least a first and a second part, and then either of the first and second part or even both can be diluted prior to introduction into the fluidic channel. In such instances the diluent for the first and second part of the blood sample can be the same or different.

According to certain embodiments at least the second part of the blood sample introduced in step b1-ii) or step b2-ii b) is diluted. For steps b1-ii) and steps b2-ii b) it is particularly preferred that the dilution is to such extent that a mono layer of settled cells is achieved in step b1-iii) or b2-iii b).

The dilution of the blood sample, or at least of the first and/or second part of the blood sample, is not particularly restricted, and dilution can be carried out using dilution liquids / diluents usually used for diluting blood samples, e.g. a PBS (phosphate buffered saline) solution. However, also other solutions can be used for dilution, e.g. media that lead to a changed, particularly increased, diffraction index between cells on the one hand and the further blood sample and the medium on the other hand, in order to increase an optical "contrast" between the cells and the further components of the diluted sample. An example is e.g. the use of Fast Green FCF in a solution, wherein a suitable concentration may be e.g. 20 mmol to 30 mmol, which can be e.g. applied to a suitably adapted PBS solution, wherein particularly osmolarity of the dilution solution, e.g. a buffer solution, has to be considered (e.g. adults and children: 275-296 mosmol/kg H₂O; infants 260-275 mosmol/kg H₂O).

The dilution of the blood sample can be suitably carried out by the skilled person, e.g. depending on the dimensions of the fluidic channel, and the skilled person can suitably select dilution based on simple calculations. For example, for a rectangular fluidic channel with a length of 10 to 40 mm with a cross section of 100 µm x 2000 µm a dilution with a PBS solution of 1:100 to 1:400 may be suitable, depending e.g. also on hematocrit, respectively symptoms of a patient, in order to achieve a mono layer. For a quick estimate in a microchannel, where the mono layer formation is predominately depending on channel height, the mono layer also may just be suitably achieved with a dilution that can be calculated by: height of channel (in µm) multiplied with 1 to 4.

According to certain embodiments, the dilution for the first and/or second part of the blood sample introduced in step b1-ii) or step b2-ii b) is at least 1:40, e.g. at least 1:50, e.g. at least 1:70, e.g. at least 1:100, e.g. at least 1:400, in an aqueous solution, particularly in a PBS (phosphate buffered saline) solution, particularly having an NaCl concentration of 137 mmol/L, a KCl concentration of 2.7 mmol/L, a Na2HPO4 concentration of 10 mmol/L and a KH2PO4 concentration of 1.8 mmol/L in distilled water.

According to certain embodiments, also the first part of the blood sample introduced in step a-ii) is diluted, e.g. in an aqueous solution, particularly in a PBS solution. According to certain embodiments, the dilution for the first part of the blood sample introduced in step a-ii is at least 1:40, e.g. at least 1:50, e.g. at least 1:70, e.g. 1:100, e.g. at least 1:400, in an aqueous solution, particularly in a PBS (phosphate buffered saline) solution, particularly having an NaCl concentration of 137 mmol/L, a KCl concentration of 2.7 mmol/L, a Na2HPO4 concentration of 10 mmol/L and a KH2PO4 concentration of 1.8 mmol/L in distilled water.

In case a dilution is carried out, according to certain embodiments also reference measurements are made in the analyzing steps with the pure dilution liquid so that the influence of the dilution liquid can be considered and taken away using suitable calculation in the analysis.

Further, the focusing of the cells of the first part of the blood sample inside the fluidic channel into one region of the fluidic channel in step a-iii) is not particularly restricted. This can be done e.g. by suitably separating the cells from the further plasma, etc. in a flown manner, e.g. using e.g. a sheath flow or parallel flow with mixing elements, etc., or in a stopped flow and/or a flown manner, preferably a stopped flow, e.g. using a cell focusing means like acoustophoresis. If a flow is provided, this can e.g. be provided by the respective introducing element, e.g. the first introducing element. For focusing, a suitable focusing unit can be provided, e.g. a unit for providing a sheath flow or parallel flow, e.g. a parallel entry and exit port with a separate parallel introducing element, and mixing elements, e.g. structures, side flows, etc., or an acoustophoretic device comprising an acoustic transducer e.g. suitably bonded to an outer surface of the fluidic channel, the acoustic transducer configured to generate, e.g. ultrasonic, acoustic standing waves inside the blood sample. Particularly, using acoustophoresis, a suitable focusing is possible, and the occurrence of cells in the region different from the region where the cells are focused in step a-iv) can be minimized. A suitable focusing level in this case can be set by the skilled person, depending e.g. on fluidic channel dimensions. When a certain energy input is exceeded, respectively starting from a certain frequency, cells start to lyse and will not only move. For example, in a microfluidic channel with a cross section of 100 µm x 2000 µm cell focusing is achieved at 341 kHz and 25 Vpp (peak-to-peak voltage) sinus excitation, whereas at 361 kHz and 50 Vpp sinus excitation cell lysing starts.

However, it is not excluded that cells are present in a region outside the focusing region inside the fluidic channel, but preferably at least a cell-free region is generated inside the fluidic channel in a region different from the region where the cells are focused.

According to certain embodiments the focusing of the cells of the first part of the blood sample inside the fluidic channel is carried out using acoustophoresis. For this purpose, e.g. an acoustic device comprising an acoustic transducer can be used that is bonded to the outer surface of the sample vessel, particularly to form a monolithic structure, the acoustic transducer configured to generate, e.g. ultrasonic, e.g. including frequencies of 20 kHz to 20 MHz, acoustic standing waves inside the blood sample in the microchannel. Acoustophoresis therein can achieve moving of cell-sized objects into the nodes of an ultrasound-generated standing wave, as e.g. described by Grenvall, C. (2014), Acoustic Forces in Cytometry and Biomedical Applications: Multidimensional Acoustophoresis, Department of Biomedical Engineering, Lund university; Petersson, C. et al., Acoustofluidic hematocrit determination, Analytica Chimica Acta, Volume 1000, 2018, Pages 199-204, both of which are incorporated herein with regard to moving of objects, particularly cells, using acoustophoresis.

The region where the cells of the first part of the blood sample inside the fluidic channel are focused is not particularly restricted, but is preferably upstream from the region different from the region where the cells are focused used in the analyzing in step a-iv) with respect to the introduced flow of the in step a-ii), irrespective of whether step a-iii) is carried out in stopped flow or not, i.e. nearer to an injection region where the first part of the blood sample is introduced into the fluidic channel.

It is not excluded that more than one region where the cells of the first part of the blood sample inside the fluidic channel are focused is created inside the fluidic channel, as can be e.g. carried out using acoustophoresis with a standing wave inside the fluidic channel.

An acoustic transducer can selectively generate acoustic forces inside the fluidic channel which are directed to e.g. surfaces thereof, e.g. walls, inside a suitable sample vessel. An acoustic transducer can be tuned so as to provide a magnitude and/or frequency of acoustic forces so as to facilitate separation of the, preferably undamaged, blood cells from the further components of the blood sample, particularly plasma and damaged blood cells. The magnitude and/or frequency of the acoustic forces generated by the acoustic transducer can be suitably selected and are e.g. depending upon a size shape of the fluidic channel, and/or the composition of the blood sample. The acoustic transducer is not particularly restricted and can be e.g. a piezoelectric element, e.g. the acoustic transducer may be a piezoelectric ultrasonic transducer.

If an acoustophoretic device comprising an acoustic transducer is used, it is preferred that at least a part of the sample vessel surrounding the fluidic channel is constructed of a material through which acoustic waves generated by the acoustic transducer can pass, e.g. glass, crystallic materials, etc. In other regions, other materials can be used for the sample vessel.

When a standing wave is generated inside the fluidic cannel, this causes the blood cells inside to move to at least one region of the fluidic channel wherein a node of the standing acoustic wave is formed. Of course, depending on the standing acoustic wave, blood cells may also be concentrated in more than one region, e.g. depending on the number of nodes of the standing acoustic wave. Other regions outside the region where the blood cells are concentrated can then be substantially free of blood cells or even free of blood cells, i.e. form cell-free regions. In such regions then an analysis of the further components of the first part of the blood sample is possible.

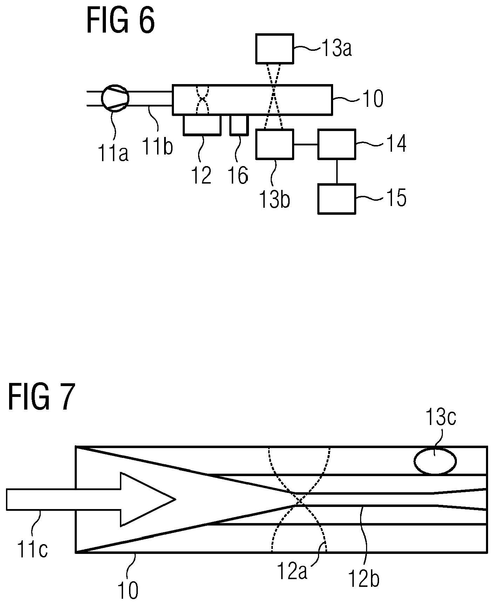

An exemplary step a-iii) of focusing cells of the first part of the blood sample inside the fluidic channel into one region of the fluidic channel is shown in Fig. 7. This step is a pre-analytical step. In this step, as shown exemplary in Fig. 7, an area of the fluidic channel 10 is created free of red blood cells by focusing the flow of the blood cells inside the introduced first part of the blood sample 11c by a standing acoustic wave 12a into one region 12b of the fluidic channel. This results in a region different from the region where the cells are focused, wherein an analysis can be carried out in step a-iv) - a suitable spot for analysis 13c shown in Fig. 7. As seen from Fig. 7, not the whole region different from the region where the cells are focused has to be used for analysis in step-iv), as long as the analysis of the first part of the blood sample in the fluidic channel is carried out in a region different from the region where the cells are focused (leaving the possibility for regions where neither the cells are focused nor an analysis in step a-iv) is carried out.

In the present method, further the step a-iv) of analyzing the first part of the blood sample in the fluidic channel in a region different from the region where the cells are focused, particularly a cell-free region, wherein analysis includes measuring at least one property w of the blood sample, particularly at least one of extracellular hemoglobin content x, extracellular lipid content y, and maximum length of clots z, is not particularly restricted. The region different from the region where the cells are focused is not particularly restricted, but is preferably downstream - as seen from the location of introduction - from the region where the cells are focused. Particularly, the region is chosen such that no red blood cells, and particularly no blood cells, are present, as these may influence the measurement of the at least one property w of the blood sample. For example, in a 2000 µm wide microchannel, such areas can include a width of up to 500 µm on both sides, depending on the setup. However, it is not excluded that a few cells are present in the region if they do not negatively influence the measurement, e.g. less than 10 cells, preferably less than 5 cells, particularly 2 cells or less or even only 1 cell, and/or at least no red blood cells are present. If isolated cells are present in the measurement region, these can be suitably subtracted out using suitable calculations known to the skilled person.

In the analysis in step a-iv), it is preferred to measure at least one of extracellular hemoglobin content x, extracellular lipid content y, and maximum length of clots z, and preferably at least two of those, e.g. extracellular hemoglobin content x and extracellular lipid content y, and particularly all three, i.e. extracellular hemoglobin content x, extracellular lipid content y, and maximum length of clots z. It is, however, not excluded that further properties of the first part of the blood sample are measured, e.g. a bilirubin content, and/or urea with measurement in the UV region.

The measurement of the at least one property w of the blood sample, particularly at least one of extracellular hemoglobin content x, extracellular lipid content y, and maximum length of clots z can be done using any suitable measurement method. For example, the at least one property w of the blood sample, particularly at least one of extracellular hemoglobin content x and extracellular lipid content y, but also e.g. a bilirubin content, can be done using a spectroscopic method, which is not particularly limited, e.g. using UV-Vis spectroscopy. For example, values for extracellular hemoglobin content x, extracellular lipid content y and bilirubin can be obtained using absorption measurement at about 410 nm, about 630 nm and about 450 nm, respectively, where the respective contents can then be suitably calculated. Measurement of maximum length of clots z is also not particularly restricted and can be e.g. done using an imaging, e.g. microscopic, method. According to certain embodiments, analyzing the first part of the blood sample in the fluidic channel in a region different from the region where the cells are focused in step a-iv) is carried out using a spectroscopic and/or microscopic method. The measurement can be, according to certain embodiments, carried out in a device for analyzing a blood sample of the present invention using a first analyzing unit configured to analyze the first part of the blood sample in a region different from the region where the cells are focused in step a-iv), e.g. a microscope and/or a spectrometer, e.g. UV-Vis spectrometer.

In this regard, it is also not excluded to use the same analysis method is also used in a following step, e.g. in one or more of the steps of b1-iv) optionally analyzing the settled cells at least for cell types, b1-vi) analyzing at least the lysed red blood cells, b2-iv b) optionally analyzing the settled cells at least for cell types and b2-vi b) analyzing at least the lysed red blood cells, although for these steps also different analysis methods and/or analysis components can be used. The analysis of the first part of the blood sample in the fluidic channel in a region different from the region where the cells are focused, particularly a cell-free region, is exemplary also shown in Fig. 7, as described above, in a suitable spot for analysis 13c.

Thus, particularly, in the cell-free, particularly red-cell-free, region, a measuring of the transmittance of different wavelengths of light, taking into account the different extinction coefficients of lipids, hemoglobin and other plasma components, and/or a microscopic imaging of blood components like clots, can be carried out, and an extracellular hemoglobin and/or lipid concentration and/or a maximum length of clots can be derived.

In the method of analyzing a blood sample of the present invention, also the step a-v) of automatically outputting at least a preliminary result of the analysis of the first part of the blood sample, wherein the preliminary result includes the at least one property w of the blood sample, particularly at least one result of an extracellular hemoglobin content x, an extracellular lipid content y, and a maximum length of clots z, is not particularly restricted, and the automatically outputting can be done at any location, e.g. on and/or near a device for analyzing a blood sample, particularly the one according to the invention, and/or at a remote location, e.g. a monitor of a medical personal, etc.

The automatically outputting therein can also include providing the at least preliminary result of the analysis of the first part of the blood sample, wherein the preliminary result includes the at least one property w of the blood sample, particularly at least one result of an extracellular hemoglobin content x, an extracellular lipid content y, and a maximum length of clots z, with a suitable marker, e.g. a flag of any color, in case the at least one property w of the blood sample is above a reference value, particularly if the result for extracellular hemoglobin content x is above a value of 150 mg/dL, and/or the result of extracellular lipid content y is above a value of 600 mg/dL, and/or the maximum length of clots z is more than 20 µm.

It is not excluded to use also different markers, e.g. different flags and/or different colors, are used depending on the measurement result, e.g. using a yellow marker, e.g. a yellow flag, if the result of the at least one property w of the blood sample is above a reference value, e.g. w1, and equal to or below double the reference value, e.g. 2 x w1, particularly if the result for extracellular hemoglobin content x is above a value of 150 mg/dL and equal to or below 300 mg/dL, particularly equal to or below 200 mg/dL, and/or the result of extracellular lipid content y is above a value of 600 mg/dL and equal to or below 1200 mg/dL, particularly equal to or below 800 mg/dL, and/or the maximum length of clots z is more than 20 µm and equal to or less than 40 µm, particularly equal to or less than 25 µm, and use a different marker, e.g. a red marker, e.g. a red flag, if the result of the at least one property w of the blood sample is above double the reference value, e.g. 2 x w1, particularly if the result for extracellular hemoglobin content x is above a value of 300 mg/dL, and/or the result of extracellular lipid content y is above a value of 1200 mg/dL, and/or the maximum length of clots z is above 40 µm. In such case, e.g. an orange marker, e.g. an orange flag, can be used for values inbetween, e.g. for a value of the at least one property w of the blood sample equal to or above a value w2 (w2 > w1) and below double the reference value, e.g. 2 x w1, e.g. if the result for extracellular hemoglobin content x is above a value of 200 mg/dL and equal to or below a value of 300 mg/dL, and/or the result of extracellular lipid content y is is above a value of 800 mg/dL and equal to or below a value of 1200 mg/dL, and/or the maximum length of clots z is more than 25 µm and equal to or below 40 µm. If the result of the at least one property w of the blood is equal to or below a reference value, particularly if the result for extracellular hemoglobin content x is equal to or below a value of 150 mg/dL, and/or the result of extracellular lipid content y is equal to or below a value of 600 mg/dL, and/or the maximum length of clots z is equal to or less than 20 µm, it is not necessary to show a marker, e.g. a flag, but it is also not excluded to show a marker, e.g. a green marker, e.g. a green flag.

If a marker, e.g. a flag, is shown, it is in some cases also not necessary to directly show the at least a preliminary result of the analysis of the first part of the blood sample, wherein the preliminary result includes the at least one property w of the blood sample, particularly at least one result of an extracellular hemoglobin content x, an extracellular lipid content y, and a maximum length of clots z at the time of or directly after step a-iv) of analyzing the first part of the blood sample in the fluidic channel in a region different from the region where the cells are focused. The preliminary results in such case may also be shown together with further results in a later step, e.g. an optional step b1-vii) of, preferably automatically, outputting results of step b1-iv) of optionally analyzing the settled cells at least for cell types and/or step b1-vi) of analyzing at least the lysed red blood cells; and/or an optional step b2-viii b) of, preferably automatically, outputting results of step b2-iv b) of optionally analyzing the settled cells at least for cell types and/or step b2-vi b) of analyzing at least the lysed red blood cells - which can also be included and/or at the same time as step b2-vii b) of outputting a result wherein it is indicated that the result may be erroneous; and/or the preliminary results may be shown in step b2-ii a) of automatically outputting a result that the blood sample is to be discarded and a new blood sample may need to be provided or in step b2-vii b) of outputting a result wherein it is indicated that the result may be erroneous.

As described, after step a-v), either steps b1-i) to b1-vi) and optionally further steps, e.g. step b1-vii); steps b2-i a) and b2-ii a) and optionally further steps; or step b2-i b) to b2-vii b) and optionally further steps, e.g. step b2-viii b), are carried out.

In all of these steps, the first step, i.e. step b1-i), b2-i a) and b2-i b), involves a flushing of the first part of the blood sample from the fluidic channel. The flushing in either of these steps can be the same or different and is not particularly restricted. Flushing can be done using any suitable liquid and/or gas, particularly liquid, e.g. distilled water; dilution liquids / diluents usually used for diluting blood samples, e.g. a PBS (phosphate buffered saline) solution or others, e.g. mentioned above; a further part of the blood sample which can also be diluted, e.g. as above, or another suitable liquid. Particularly preferable is flushing using a further part of the blood sample which can also be diluted and have the same composition as the first and/or second part of the blood sample, so that a source of uncertainty due to e.g. diluting effects can be further minimized. Flushing can e.g. be, according to certain embodiments, carried out using a first introducing element configured to introduce a first part of the blood sample into the fluidic channel, which is then also configured to flush the channel; a second introducing element configured to introduce a second part of the blood sample into the fluidic channel, which is then also configured to flush the channel; or a further separate flushing element configured to flush the first part of the blood sample from the fluidic channel, all of which can be part of the device for analyzing a blood sample of the present invention.

The amount of flushing is not particularly limited. Usually, an amount of at least five time the volume of the fluidic channel, and/or up to 20 times or at least up to ten times of the volume of the fluidic channel, particularly in case of a microchannel, is used

Depending on the results, the first and third variant are then quite similar in the following steps, which will be discussed in detail in the following.

In the first variant the step b1-ii) of introducing a second part of the blood sample into the fluidic channel and in the third variant step b2-ii b) of introducing a second part of the blood sample into the fluidic channel can be the same or different and are not particularly restricted. The second part of the blood sample can be diluted or undiluted, as the first part of the blood sample, and is preferably diluted. The dilution of the second part of the blood sample can be the same as the one of the first blood sample, i.e. dilution can be carried out using dilution liquids / diluents usually used for diluting blood samples, e.g. a PBS (phosphate buffered saline) solution, and again also other solutions can be used for dilution, e.g. media that lead to a changed, particularly increased, diffraction index between cells on the one hand and the further blood sample and the medium on the other hand, in order to increase an optical "contrast" between the cells and the further components of the diluted sample.

According to certain embodiments, the dilution for the second part of the blood sample introduced in step b1-ii) or step b2-ii b) is at least 1:40, e.g. at least 1:50, e.g. at least 1:70, e.g. 1:100, in an aqueous solution, particularly in a PBS (phosphate buffered saline) solution, particularly having an NaCl concentration of 137 mmol/L, a KCl concentration of 2.7 mmol/L, a Na2HPO4 concentration of 10 mmol/L and a KH2PO4 concentration of 1.8 mmol/L in distilled water.

The introduction of the second part of the blood sample can be done with the same introducing element as the first part of the blood sample or be different, e.g. can be done by the first introducing element of a device of the present invention, configured to also introduce a second part of the blood sample into the fluidic channel, or by a second introducing element configured to introduce a second part of the blood sample into the fluidic channel. As discussed above, the second part of the blood sample can be introduced from a syringe or a similar device, a reservoir, e.g. also on a chip, e.g. microchip that also contains the fluidic channel, etc., via a tube, separate channel, etc., using a pump, an automatic device for pressing the syringe, etc. The syringe with a tube, the reservoir with e.g. a tube and a pump, etc. therein then can form a second introducing element.

Step b1-iii) in the first variant of settling cells of the second part of the blood sample inside at least a part of the fluidic channel and step b2-iii b) in the third variant of settling of cells of the second part of the blood sample inside at least a part of the fluidic channel can be the same or different and are not particularly restricted. Particularly, at least red blood cells are settled. Settling of the cells can be e.g. carried out in a stopped flow, i.e. by stopping the flow and letting the cells settle on at least one surface of the fluidic channel. Settling can be done any other way, though, e.g. also by focusing cells as in step a-iii) .

Furthermore, step b1-iv) in the first variant of optionally analyzing the settled cells at least for cell types and step b2-iv b) in the third variant of optionally analyzing the settled cells at least for cell types is not particularly restricted, and both steps can be the same or different. In the first variant preferably step b1-iv) is carried out, and in the third variant preferably step b2-iv b) is carried out. Analyzing the settled cells can be done using a suitable measurement method, e.g. a spectroscopic and/or microscopic method. Step b1-iv) in the first variant and step b2-iv b) in the third variant can, according to certain embodiments, be carried out by at least a first analyzing unit configured to analyze cells of the blood sample and/or products of a cell lysis of at least red blood cells of the blood sample in the present device for analyzing a blood sample, the first analyzing unit also configured to analyze the first part of the blood sample in a region different from the region where the cells are focused in step a-iv), and/or at least a second analyzing unit configured to analyze the cells of the blood sample and/or products of a cell lysis of at least red blood cells of the blood sample. If both the at least first and at least second analyzing unit are present, they can be of the same type and/or of a different type, and they can analyze the first and second part of the blood sample, respectively, at the same location or at different locations of the fluidic channel.

Analyzing the settled cells is not particularly restricted and can be done using usual methods for cell analysis, e.g. microscopy and/or spectroscopy, e.g. for determining cell type, cell volume - particularly for red blood cells (RBC), and/or intracellular hemoglobin. For example, thus the first and/or second analyzing unit can comprise a microscope and/or spectroscope, e.g. a UV-Vis spectroscope.

In these analytical steps b1-iv) and b2-iv b), in the same fluidic channel without a need for tube movement or new sample aspiration, hematological parameters can be determined by the optical readout of blood cells through any microscopy or localized spectroscopy method of choice. Intracellular quantitative values can be determined, e.g. intracellular hemoglobin and hematocrit / RBC count (forming part of a standard hematology analysis).

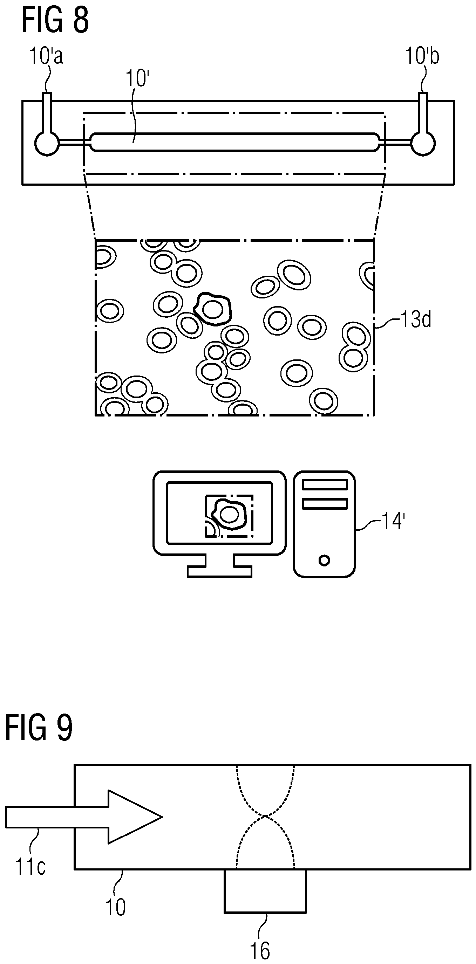

An exemplary setup for these steps is shown in Fig. 8, showing an exemplary microchannel 10' as fluidic channel with an entry port 10'a and an exit port 10'b, wherein a spot for blood cell analysis 13d observed with a microscope is shown, which can be visualized using a suitable outputting interface, e.g. a screen 14' of a computer.

Step b1-v) in the first variant of lysing at least red blood cells of the settled cells and step b2-v b) in the third variant of lysing at least red blood cells of the settled cells can also be the same or different and are not particularly restricted. It is therein not excluded that also other cells are lysed as well, but at least red blood cells are lysed, as this can achieve e.g. analysis of total hemoglobin in the blood sample.

Lysing can be done by applying e.g. a suitable shear force on the cells and/or by applying suitable agents for cell lysis, e.g. ammonium chloride buffer solution, e.g. using a further flow applied upon settling the cells. While it may be possible to apply shear through flow, e.g. if the settled cells are somehow fixed in the fluidic channel, e.g. using structures inside the fluidic channel, or in other ways, according to certain embodiments the lysing of at least the red blood cells is carried out using ultrasonic waves. For this purpose, an acoustophoretic device, e.g. comprising an acoustic transducer, can be used that is bonded to the outer surface of the sample vessel, particularly to form a monolithic structure, the acoustic transducer configured to generate ultrasonic acoustic standing waves inside the blood sample in the fluidic channel, e.g. a microchannel. According to certain embodiments, the acoustic transducer is a piezoelectric ultrasonic transducer.

For step b1-v) in the first variant and step b2-v b) in the third variant, the same acoustophoretic device, e.g. comprising an acoustic transducer, can be used - according to certain embodiments - as in step a-iii), a difference in the frequency used for lysing the cells which the skilled person can suitably select and/or which can be automatically obtained from the hardware in connection with software available to the skilled person. As stated above, for example, in a microfluidic channel with a cross section of 100 µm x 2000 µm cell focusing is achieved at 341 kHz and 25 Vpp sinus excitation, whereas at 361 kHz and 50 Vpp sinus excitation cell lysing starts.

For this purpose, the device may also contain a controller configured to control the acoustophoretic device such that suitable acoustic waves, e.g. ultrasonic waves, are applied in step a-iii) and in step b1-v) in the first variant and step b2-v b) in the third variant. However, it is not excluded that in step b1-v) in the first variant and step b2-v b) in the third variant a different, further (second) acoustophoretic device is used as in step a-iii).

The acoustophoretic device, e.g. comprising the acoustic transducer, can be configured to bend the sample vessel such that shear forces are induced within the fluidic channel, e.g. a microchannel, the acoustic standing waves and the shear forces causing cavitation in the blood sample thereby rupturing cell walls in the blood sample. For example, when a sample vessel comprising a fluidic channel, e.g. a microchannel, is made of glass, the microchannel having a width of approximately two millimeters with an aspect ratio of 0.05 to 0.125, and the sample vessel having a width of approximately twelve millimeters with an aspect ratio of 1.4 to 1.9, the acoustic transducer may be configured to produce ultrasonic sound waves in the range of 330 kHz to 350 kHz with peak pressure within the microchannel of five MPa, and peak velocity up to eight m/s.

According to certain embodiments the lysing of at least the red blood cells is carried out in stopped flow. This enables good control of lysing as well as of products of the lysing for later analysis. In addition, also the influx of further cells that may affect the results, e.g. increase the Hb value (hemoglobin value) can be avoided.

An exemplary cell lysis using, for example, ultrasonic vibrations utilizing a different setting on the same acoustophoretic device as used for the pre-analytical cell separation in step a-iii), is shown in Fig. 9, where upon introduction of the second part of blood sample 11c in the fluidic channel 10 and settling of cells (not shown) a standing acoustic wave is generated by a lysing unit 16, e.g. an acoustophoretic device for lysing at least the red blood cells.

Step b1-vi) in the first variant of analyzing at least the lysed red blood cells and step b2-vi b) in the third variant of analyzing at least the lysed red blood cells can be the same or different and are not particularly restricted. Analysis can be carried out using the same device as in step a-iv), e.g. a spectrometer, e.g. UV-Vis spectrometer, e.g. measuring total hemoglobin content, e.g. at a wavelength of about 410 nm, or in a different device, e.g. a different spectrometer. The determination thus can take place e.g. using the same optical transmittance method as in the pre-analytical step a-iv). After lysing, e.g. total hemoglobin can be determined, particularly using suitable calculation.

Step b1-vi) and b2-vi b) can be seen as a post-analytical step, as it allows also verification of the earlier results. In the analytical and post-analytical steps, total values (intra- plus extra-cellular) for the relevant parameters can be determined before and/or after lysis in the same fluidic channel, e.g. inside the same sample vessel, e.g. a fluidic cell, without a need for further measures like tube movement or new sample aspiration, either in a continuous flow-in of the same sample, or using cells in a stopped flow utilized in the analytical step.

According to certain embodiments, the first part of the blood sample and the second part of the blood sample can thus also be flown continuously, e.g. with also a further part of the blood sample flown in between for flushing the fluidic channel. However, at least for the second part of the blood sample at least a stopped flow at some instance, e.g. during settling of at least red blood cells, is preferred.

After step b1-vi) in the first variant can follow an optional step b1-vii) of, preferably automatically, outputting results of step b1-iv) of optionally analyzing the settled cells at least for cell types and/or step b1-vi) of analyzing at least the lysed red blood cells. This outputting of results is not particularly restricted, and the outputting can be e.g. done also together with step a-v), i.e. having step a-v) after step b1-vi), or separate.

Step b2-vii b) in the third variant of outputting a result wherein it is indicated that the result may be erroneous is not particularly restricted, and as already discussed for step a-v) the indication can be done in any way, using e.g. a suitable marker, e.g. a flag, e.g. a yellow or orange flag. While the results in the third variant are not necessarily deemed to be erroneous, the high value of the at least one property w of the blood sample, particularly the at least one high result of an extracellular hemoglobin content x and/or an extracellular lipid content y and/or the presence of clots with a maximum length of clots z that is too high is indicative that there at least may be some problems with the blood sample and that at least a verification using a different sample is advantageous. At any rate in the third variant the values of analyzing the first part of the blood sample can be saved and provided together with the analytical results of the second part of the blood sample, indicating that the sample may be erroneous, e.g. having too high interference. Together with step b2-vii b) or thereafter in the third variant (variant 3) can also be carried out an optional step b2-viii b) of, preferably automatically, outputting results of step b2-iv b) of optionally analyzing the settled cells at least for cell types and/or step b2-vi b) of analyzing at least the lysed red blood cells, which is not particularly restricted. Again, this outputting can be also carried out together with step a-v), i.e. step a-v) being carried out after step b2-vi b).

In the second variant (variant 2), step b2-ii a) of automatically outputting a result that the blood sample is to be discarded and a new blood sample may need to be provided is not particularly restricted. In the second variant, the results of the analysis of the first part of the blood sample, e.g. given certain adjustable set parameters, show that the sample testing can be terminated, and the sample can be returned as having too high interference.

After step b1-v), e.g. together or after step b1-vi) and/or step b1-vii), in the first variant (variant 1); or after step b2-ii a) in the second variant; or after step b2-v b), e.g. together or after step b2-vi b)) and/or step b2-vii b), in the third variant, can, according to certain embodiments, follow at least a step of flushing the second part of the blood sample from the fluidic channel, and the flushing is not particularly restricted. It is preferred to flush the fluidic channel with a neutral liquid, e.g. distilled water, and/or a cleaning gas, e.g. air or even preferably an inert gas like nitrogen, to have the fluidic channel ready for a new measurement. According to certain embodiments, a fluidic channel, e.g. in a suitable sample vessel, could also be discarded, e.g. after removing units for focusing, analyzing and/or lysing as well as e.g. introducing elements if it is deemed unusable after staining, due to damage, etc.

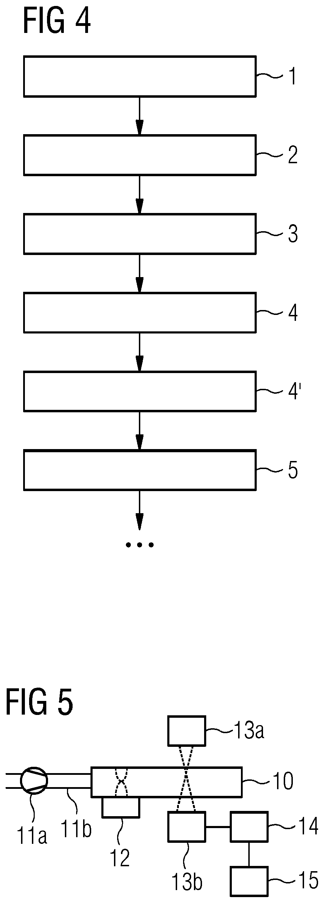

According to certain embodiments the method further comprises washing the fluidic channel after the analysis in step a-iv) with a suitable solvent and/or solution, e.g. with a further part of the blood sample, as discussed above. This is shown schematically in Fig. 4, wherein step a-iv) of analyzing the first part of the blood sample in the fluidic channel in a region different from the region where the cells are focused 4 is followed by a step of washing the fluidic channel 4'. This step can also be carried out at the same time or after step 5, although it is preferably carried out before step 5. The herein presented integrated workflow of the present method, particularly for the first and third variant in the present method can provide pre-analytical parameters, with an option to either flag (third variant) or terminate (second variant) analysis, and a post-analytical control to verify values and provide further sample information, without introducing appreciable extra cost, and only introducing a relatively low time-overhead to the test.

In a second aspect, the present invention relates to a device for analyzing a blood sample, comprising
- a fluidic channel;
- a first introducing element configured to introduce a first part of the blood sample into the fluidic channel;
- a focusing unit configured to focus cells of the first part of the blood sample inside the fluidic channel into one predetermined region of the fluidic channel;
- at least a first analyzing unit configured to analyze the first part of the blood sample in a region different from the region where the cells are focused, including measuring at least one property w of the blood sample, particularly at least one of an extracellular hemoglobin content x, an extracellular lipid content y, and a maximum length z of clots; and
- an outputting interface configured to automatically output at least a preliminary result of the analysis of the blood sample, wherein the preliminary result includes at least one property w of the blood sample, particularly at least one result of the extracellular hemoglobin content x, the extracellular lipid content y, and the maximum length z of clots, further comprising
- a decision module configured to compare a measured result of the at least one property w of the blood sample to a reference value, particularly at least one of the extracellular hemoglobin content x, the extracellular lipid content y, and the maximum length z of clots, to a reference value of 150 mg/dL of extracellular hemoglobin content, a reference value of 600 mg/dL of extracellular lipid content, and a reference value of a maximum length of clots of 20 µm, respectively, wherein
   if the result of the at least one property w of the blood sample is equal to or below the reference value, particularly if the result for extracellular hemoglobin content x is equal to or below the reference value of 150 mg/dL, and/or the result of extracellular lipid content y is equal to or below the reference value of 600 mg/dL, and/or the maximum length of clots z is equal to or less than 20 µm, the decision module is configured to provide an indication in the outputting interface that a second part of the blood sample can be measured, or is configured to provide no indication in the outputting interface, or
   if the result of the at least one property w of the blood sample is above the reference value, particularly if the result for extracellular hemoglobin content x is above a reference value of 150 mg/dL, and/or the result of extracellular lipid content y is above a reference value of 600 mg/dL, and/or the maximum length of clots z is above 20 µm, the decision module is configured to provide an indication in the outputting interface that the blood sample is to be discarded and a new blood sample may need to be provided, or that the result may be erroneous;
   further wherein the device comprises a second introducing element configured to introduce a second part of the blood sample into the fluidic channel, or wherein the first introducing element is further configured to also introduce a second part of the blood sample into the fluidic channel; and
   further wherein the device comprises a lysing unit configured to lyse at least red blood cells settled inside at least a part of the fluidic channel; or wherein the focusing unit is further configured to lyse at least red blood cells settled inside at least a part of the fluidic channel.

With the device for analyzing a blood sample of the second aspect particularly the method of analyzing a blood sample of the first aspect can be carried out. Thus, embodiments and implementations described with regard to the method of the first aspect above also may be applicable to the device of the second aspect, and vice versa.

In the present device, the fluidic channel can be set up as described for the present method. The fluidic channel is not particularly restricted, as long as it comprises a structure having inner surfaces through which the blood sample can be flown, wherein the structure is configured to contain the blood sample introduced. According to certain embodiments the fluidic channel is a microchannel, particularly in a suitable sample vessel, made from a suitable material, e.g. glass and/or a suitable polymer resin, for analysis of the blood sample, e.g. with suitable transmissivity for a spectroscopic and/or microscopic analysis for analyzing the first part of the blood sample in the fluidic channel in a region different from the region where the cells are focused, optionally analyzing the settled cells at least for cell types, and/or analyzing at least the lysed red blood cells. Alternatively, it can also be a pipe, etc., but preferably comprises at least two parallel inner and outer surfaces for focusing and/or settling cells and/or for ease of analysis. According to preferred embodiments, the fluidic channel is a microchannel in a suitable sample vessel, e.g. inside a chip made e.g. from glass, plastic, etc.

A suitable sample vessel, according to certain embodiments, has an outer surface, the fluidic channel, e.g. a microchannel, within the confines of the outer surface, a first port extending through the outer surface to the microchannel, and a second port extending through the outer surface to the microchannel, such that parts of the blood sample can be introduced through the first port into the microchannel and can be flushed from the second port.

According to certain embodiments, the fluidic channel, particularly a microchannel, has a length, a width and a height, wherein preferably an aspect ratio of the width to the height is in a range from approximately 0.04 to approximately 0.175, and/or wherein the sample vessel has a width and a height, and wherein a sample vessel aspect ratio of the width to the height is in a range from approximately 0.5 to approximately 3.0. According to certain embodiments the fluidic channel is a microchannel with a height of between and including 5 and 500 µm, preferably between and including 10 and 200 µm, further preferably between and including 50 and 100 µm.

Furthermore, the first introducing element configured to introduce a first part of the blood sample into the fluidic channel is also not particularly restricted, and can comprise e.g. a syringe or a similar device, a reservoir, e.g. also on a chip, e.g. microchip that also contains the fluidic channel, etc., a tube, separate channel, etc., using a pump, an automatic device for pressing the syringe, etc., or a combination thereof. For example, a syringe with a tube, a reservoir with e.g. a tube and a pump, etc. can form a first introducing element.

Further, if present the second introducing element configured to introduce a second part of the blood sample into the fluidic channel is also not particularly restricted, and can also comprise e.g. a syringe or a similar device, a reservoir, e.g. also on a chip, e.g. microchip that also contains the fluidic channel, etc., a tube, separate channel, etc., using a pump, an automatic device for pressing the syringe, etc., or a combination thereof. For example, a syringe with a tube, a reservoir with e.g. a tube and a pump, etc. can form a second introducing element.

Alternatively, it is of course also possible, and according to certain embodiments preferred, to introduce the first and second part of the blood sample with the same first introducing element, i.e. the first introducing element is further configured to also introduce a second part of the blood sample into the fluidic channel. In such case the first part can be introduced by the first introducing element in a step of introducing the first part of the blood sample into the fluidic channel, while keeping at least a second part in at least part of the first introducing element, e.g. a syringe or a reservoir. Then, a second part of the blood sample can be introduced at a later time in a step of introducing the second part of the blood sample into the fluidic channel, e.g. using the same mechanism as used for introducing the first part, e.g. pressure on the syringe, pumping from a reservoir, etc.

According to certain embodiments, the blood sample is pumped into the channel, preferably with an optical read-out. The optical read-out allows introducing only a suitable amount of blood sample.

According to certain embodiments, the fluidic channel comprises at least an outlet from which the first and optionally second part of the blood sample, as well as optionally further media for flushing, washing, etc. can exit the fluidic cannel, the at least one outlet not particularly restricted.

In the present device, also the focusing unit configured to focus cells of the first part of the blood sample inside the fluidic channel into one predetermined region of the fluidic channel is not particularly restricted, and can comprise e.g. further fluidic means to establish e.g. a sheath flow, like a second introduction channel, etc., contraptions inside the fluidic channel to separate the flow of the first part of a blood sample, and/or external units, e.g. using acoustic waves. According to certain embodiments the focusing unit is an acoustophoretic device, particularly comprising an acoustic transducer. According to certain embodiments, the acoustic transducer is a piezoelectric ultrasonic transducer, further details of preferred embodiments also given with regard to the present method above.

Furthermore, the at least first analyzing unit configured to analyze the first part of the blood sample in a region different from the region where the cells are focused, including measuring at least one property w of the blood sample, particularly at least one of an extracellular hemoglobin content x, an extracellular lipid content y, and a maximum length z of clots, is not particularly restricted, and can be any analytical device which enables quantitative measurement of blood components.

As described above, particularly at least one of extracellular hemoglobin content x and extracellular lipid content y, but also e.g. a bilirubin content, can be measured using a spectroscopic method, which is not particularly limited, e.g. using UV-Vis spectroscopy, and values for extracellular hemoglobin content x, extracellular lipid content y and bilirubin can be obtained using absorption measurement at about 410 nm, about 630 nm and about 450 nm, respectively. Thus, the at least first analyzing unit may comprise a spectrometer, e.g. UV-Vis spectrometer, configured to take measurement in the UV-Vis range (wavelength 200 - 800 nm), particularly in a wavelength range from 400 to 700 nm. According to certain embodiments, an exemplary first and/or second analyzing unit may comprise a spectrometer, e.g. an absorbance spectrometer, e.g. spectrophotometer, comprising a transmitter and a receiver positioned adjacent to the fluidic channel or a sample vessel comprising the fluidic channel, the transmitter positioned to emit a light medium through the fluidic channel, e.g. microchannel, and the receiver positioned to receive at least a portion of the light medium after the portion of the light medium has passed through the microchannel.

Measurement of maximum length of clots z can be e.g. done using an imaging, e.g. microscopic, method, so that the at least first analyzing unit may comprise an imaging device, e.g. a microscope, a suitable camera, and/or a linear photodiode array. The first analyzing unit can also be an integrated device, e.g. a microscope comprising suitable filters and/or LEDs, etc., so that measurements can be carried out at different wavelengths.

According to certain embodiments the at least first analyzing unit is also configured to analyze cells of the blood sample and/or products of a cell lysis of at least red blood cells of the blood sample, and/or the device further comprises at least a second analyzing unit configured to analyze the cells of the blood sample and/or products of a cell lysis of at least red blood cells of the blood sample.

The at least first analyzing unit particularly is arranged in the present device in such a way that it analyzes the first part of the blood sample in a region different from the region where the cells are focused, thus apart from the focusing unit, preferably downstream in view of an introduced blood stream in the fluidic channel.

Furthermore, the outputting interface configured to automatically output at least a preliminary result of the analysis of the blood sample is not particularly restricted, and may comprise a screen, monitor, display, etc., on which a preliminary result of the analysis of the blood sample can be displayed, and optionally also indications like markers, e.g. flags - as described with regard to the above second and third variant of the present method - and/or results of analysis of at least red blood cells of the blood sample and/or products of a cell lysis of at least red blood cells of the blood sample can be displayed. For this purpose the outputting interface may be connected to the at least first analyzing unit and optionally the decision module and/or at least a second analyzing unit, and also to a suitable processing unit for storing and/or further compiling and/or processing of the analysis data.

In the device, also the decision module configured to compare a measured result of the at least one property w of the blood sample to a reference value is not particularly restricted, as long as it can compare the measured result to a reference value, decide whether measured value is equal to or below the reference value or above the reference value, and depending on the decision provide an input to the outputting interface to indicate whether the measurement result was equal to or below the reference value or above the reference value. According to certain embodiments, the decision module is also configured, particularly in an automated measurement setup, to configure the device, depending on the measurement, for carrying out the first variant, second variant or third variant of the present method, i.e. give an input to optionally the at least first analyzing unit and/or the at least second analyzing unit, and/or the lysing unit or focusing unit, as well as e.g. a controller controlling the focusing unit in case it can be configured to focus cells of the first part of the blood sample and also to lyse at least red blood cells of the second part of the blood sample. The decision module may comprise a database comprising the reference value, or may be configured to receive a reference value from an external source.

If the present device comprises a lysing unit configured to lyse at least red blood cells settled inside at least a part of the fluidic channel, the lysing unit is not particularly restricted, and may comprise an acoustophoretic device, inlets and introducing elements configured to introduce a lysing agent, etc. Preferably the lysing unit, if present, comprises an acoustophoretic device.

According to preferred embodiments, the focusing unit is further configured to lyse at least red blood cells settled inside at least a part of the fluidic channel. In such case, the focusing unit is preferably an acoustophoretic device and further preferably the acoustophoretic device can be configured to lyse at least red blood cells of the blood sample settled inside at least a part of the fluidic channel. This means that the focusing unit can be configured to focus cells of the first part of the blood sample inside the fluidic channel into one predetermined region of the fluidic channel, and can also be configured to lyse at least red blood cells settled inside at least a part of the fluidic channel, e.g. at a later point in the above first and third variant of the present method. According to certain embodiments the acoustophoretic device is configured to lyse at least red blood cells of the blood sample settled inside at least a part of the fluidic channel.

According to certain embodiments, the device comprises a controller electrically connected to the focusing unit, particularly an acoustic transducer, configured to provide electrical signals to the acoustic transducer that when received by the acoustic transducer cause the acoustic transducer to emit e.g. ultrasonic acoustic waves and/or cause the acoustic transducer to contract and elongate. The controller may be constructed of suitable circuitry and is not particularly restricted.

According to certain embodiments the device further comprises a flow control unit configured to control a flow inside of the fluidic channel. The flow unit particularly is configured to control the flow to be continuous or stopped.

A first exemplary device of the present invention is schematically shown in Fig. 5. From the first introducing element 11a/b, comprising e.g. a pump 11a and a tube 11b, the first and second part of the blood sample can be introduced in fluidic channel 10, wherein cells of the first part of the blood sample can be focused using focusing unit 12. The focusing unit 12 also can in this exemplary embodiment be configured to lyse at least red blood cells of the second part of the blood sample. The further components of the first part of the blood sample, settled cells of the second part of the blood sample and products of lysing at least the red blood cells can be analyzed using the first analyzing unit 13a/b, comprising a transmitter 13a and a receiver 13b in this embodiment. The results are shown on outputting interface 14, and a decision module 15 can analyze and optionally indicate whether the sample may be erroneous or not.

In a second exemplary device shown schematically in Fig. 6 additionally a lysing unit 16 is present, and the focusing unit 12 is not configured to lyse at least red blood cells. Otherwise the setup is the same as in Fig. 5.

A third aspect of the present invention is directed to a non-transient computer program product, configured to be used in a device of the present invention for analyzing a blood sample, wherein the non-transient computer program product is configured to carry out the following steps:
a-ii) introducing a first part of the blood sample into a fluidic channel;
a-iii) focusing cells of the first part of the blood sample inside the fluidic channel into one region of the fluidic channel;
a-iv) analyzing the first part of the blood sample in the fluidic channel in a region different from the region where the cells are focused, particularly a cell-free region, wherein analysis includes measuring at least one property w of the blood sample, particularly at least one of extracellular hemoglobin content x, extracellular lipid content y, and maximum length of clots z;
a-v) automatically outputting at least a preliminary result of the analysis of the first part of the blood sample, wherein the preliminary result includes the at least one property w of the blood sample, particularly at least one result of an extracellular hemoglobin content x, an extracellular lipid content y, and a maximum length of clots z,
wherein if the result of the at least one property w of the blood is equal to or below a reference value, particularly if the result for extracellular hemoglobin content x is equal to or below a value of 150 mg/dL, and/or the result of extracellular lipid content y is equal to or below a value of 600 mg/dL, and/or the maximum length of clots z is equal to or less than 20 µm, the following steps are carried out: b1-i) flushing the first part of the blood sample from the fluidic channel;
b1-ii) introducing a second part of the blood sample into the fluidic channel;
b1-iii) settling cells of the second part of the blood sample inside at least a part of the fluidic channel;
b1-iv) optionally analyzing the settled cells at least for cell types;
b1-v) lysing at least red blood cells of the settled cells; and
b1-vi) analyzing at least the lysed red blood cells; or
wherein if the result of the at least one property w of the blood sample is above a reference value, particularly if the result for extracellular hemoglobin content x is above a value of 150 mg/dL, and/or the result of extracellular lipid content y is above a value of 600 mg/dL, and/or the maximum length of clots z is more than 20 µm, the following steps are carried out:
b2-i a) flushing the first part of the blood sample from the fluidic channel;
b2-ii a) automatically outputting a result that the blood sample is to be discarded and a new blood sample may need to be provided; or
b2-i b) flushing the first part of the blood sample from the fluidic channel;
b2-ii b) introducing a second part of the blood sample into the fluidic channel;
b2-iii b) settling of cells of the second part of the blood sample inside at least a part of the fluidic channel;
b2-iv b) optionally analyzing the settled cells at least for cell types;
b2-v b) lysing at least red blood cells of the settled cells;
b2-vi b) analyzing at least the lysed red blood cells, and
b2-vii b) outputting a result wherein it is indicated that the result may be erroneous.

The steps carried out by the non-transient computer program product are particularly the corresponding steps in the present method, and embodiments and implementations described with regard to the method of the first aspect above also thus particularly apply to the present non-transient computer program product, and vice versa.

The non-transient computer program product is not particularly restricted, and may be a suitable storage unit, e.g. a disc, USB unit, processing unit, etc., comprising code that, when executed, carry out the corresponding steps.

### Examples

The present invention will now be described in detail with reference to several examples thereof. However, these examples are illustrative and do not limit the scope of the invention.

A blood sample from a patient was provided. Further, also a fluidic system including a microchannel as fluidic channel was provided, to which the sample was provided through an inlet in separate parts.

For a pre-analytical test, extracellular hemoglobin and lipid concentration as well as maximum length of clots for clogging detection were measured. For this purpose, the fluidic channel and also other parts of the system were washed with buffer, e.g. PBS buffer as described above, and a background measurement was taken as reference. As a first part of the blood sample, 100µl whole blood sample were pumped into the microchannel trough a fluidic sample port, and the flow was stopped. Through a piezoelectric transducer attached to the microchip comprising the microchannel, an acoustic force was turned on at a specified "focusing" level of 341 kHz at 25 Vpp sinus with a microchannel having a width of 2000 µm, a height of 100 µm and length of 17 mm (outer dimensions 22 mm x 12 mm x 1.8 mm), and cells of the first part of the blood sample are focused in the middle of the channel, with a cell-free, plasma enriched region generated at the sidewalls Using a UV-Vis spectrometer, transmittance values for extracellular hemoglobin content x, extracellular lipid content y and bilirubin were obtained using absorption measurement at wavelengths of 410 nm, 630 nm and 450 nm, respectively in the cell-free region, and the maximum length of clots was determined using a microscope. The acoustic force was turned off, and the fluidic system including the microchannel was washed with buffer. The obtained results are compared to reference values, which were a value of 150 mg/dL for extracellular hemoglobin content x, 600 mg/dL for extracellular lipid content y and 20 µm for maximum length of clots z. For the blood sample the measured values were below these values of x, y and z.

For the main analysis, measurement was carried out of cellular level hemoglobin/ volume of red blood cells (RBC), and an RBC count and hematocrit measurement were carried out. A second part of the blood sample was prepared with 100µl at a suitable dilution (e.g. 1:200) of the blood sample in the buffer, and pumped to the microchannel through the fluidic sample port. Again, the flow was stopped, and it was waited until blood cells have sedimented to the bottom of the microchannel. A suitable region of the sedimented cells was imaged with microscopic illumination contrast of choice and a spectroscopic readout was taken at localized regions.

For the post-analytical test, the total hemoglobin and oxygenation state were measured. For this purpose, the piezoelectric transducer was set to a specified "lysis" level of 361 kHz at 50 Vpp sinus in the given channel and turned, so that sedimented red blood cells are lysed and homogenized. After lysis, the acoustic force is turned off, and transmittance is measured at suitable wavelengths. Finally, the fluidic system is washed with buffer for the next sample.

With the present method and device, a single sample draw of a blood sample can allow for a pre-analysis, analysis and control, including a fast and accurate pre-analysis. The device allows an integrated automatable workflow, and single detector technology and sample handling for all steps, thus streamlining hardware. Furthermore, only a low sample volume is needed, and in particularly preferred embodiments also no additional reagents apart from usually employed diluents.

With the present method and device, in-line separation of major blood cells from plasma, reading plasma and whole blood with the same optical system is possible, and particularly a microfluidic system with low volume requirements can be used for all steps, and the same hardware components and acoustophoretic device can be used for separation and lysis.

### List of reference signs

- 1: providing a blood sample obtained from a patient
- 2: introducing a first part of the blood sample into a fluidic channel
- 3: focusing cells of the first part of the blood sample inside the fluidic channel into one region of the fluidic channel
- 4: analyzing the first part of the blood sample in the fluidic channel in a region different from the region where the cells are focused
- 4': washing the fluidic channel
- 5: automatically outputting at least a preliminary result of the analysis of the first part of the blood sample
- 6: flushing the first part of the blood sample from the fluidic channel
- 7: introducing a second part of the blood sample into the fluidic channel
- 7': automatically outputting a result that the blood sample is to be discarded and a new blood sample may need to be provided
- 7": outputting a result wherein it is indicated that the result may be erroneous
- 8: settling cells of the second part of the blood sample inside at least a part of the fluidic channel
- 8a: analyzing the settled cells at least for cell types
- 9: lysing at least red blood cells of the settled cells
- 9a: analyzing at least the lysed red blood cells
- 10: fluidic channel
- 10': microchannel
- 10'a: entry port
- 10"b: exit port
- 11a: pump
- 11b: tube
- 11c: blood sample
- 12: focusing unit
- 12a: standing acoustic wave
- 12b: one region of the fluidic channel
- 13a: transmitter
- 13b: receiver
- 13c: spot for analysis
- 13d: spot for blood cell analysis
- 14: outputting interface
- 14': screen
- 15: decision module
- 16: lysing unit

## Claims

1. A method of analyzing a blood sample, comprising
a-i) providing a blood sample obtained from a patient;
a-ii) introducing a first part of the blood sample into a fluidic channel;
a-iii) focusing cells of the first part of the blood sample inside the fluidic channel into one region of the fluidic channel;
a-iv) analyzing the first part of the blood sample in the fluidic channel in a region different from the region where the cells are focused, particularly a cell-free region, wherein analysis includes measuring at least one property w of the blood sample, particularly at least one of extracellular hemoglobin content x, extracellular lipid content y, and maximum length of clots z;
a-v) automatically outputting at least a preliminary result of the analysis of the first part of the blood sample, wherein the preliminary result includes the at least one property w of the blood sample, particularly at least one result of an extracellular hemoglobin content x, an extracellular lipid content y, and a maximum length of clots z,
wherein if the result of the at least one property w of the blood is equal to or below a reference value, particularly if the result for extracellular hemoglobin content x is equal to or below a value of 150 mg/dL, and/or the result of extracellular lipid content y is equal to or below a value of 600 mg/dL, and/or the maximum length of clots z is equal to or less than 20 µm, the following steps are carried out:
b1-i) flushing the first part of the blood sample from the fluidic channel;
b1-ii) introducing a second part of the blood sample into the fluidic channel;
b1-iii) settling cells of the second part of the blood sample inside at least a part of the fluidic channel;
b1-iv) optionally analyzing the settled cells at least for cell types;
b1-v) lysing at least red blood cells of the settled cells; and
b1-vi) analyzing at least the lysed red blood cells; or
wherein if the result of the at least one property w of the blood sample is above a reference value, particularly if the result for extracellular hemoglobin content x is above a value of 150 mg/dL, and/or the result of extracellular lipid content y is above a value of 600 mg/dL, and/or the maximum length of clots z is more than 20 µm, the following steps are carried out:
b2-i a) flushing the first part of the blood sample from the fluidic channel;
b2-ii a) automatically outputting a result that the blood sample is to be discarded and a new blood sample may need to be provided; or
b2-i b) flushing the first part of the blood sample from the fluidic channel;
b2-ii b) introducing a second part of the blood sample into the fluidic channel;
b2-iii b) settling of cells of the second part of the blood sample inside at least a part of the fluidic channel;
b2-iv b) optionally analyzing the settled cells at least for cell types;
b2-v b) lysing at least red blood cells of the settled cells;
b2-vi b) analyzing at least the lysed red blood cells, and
b2-vii b) outputting a result wherein it is indicated that the result may be erroneous.

2. The method of patent claim 1, wherein the focusing of the cells of the first part of the blood sample inside the fluidic channel is carried out using acoustophoresis.

3. The method of any one of the preceding patent claims, wherein analyzing the first part of the blood sample in the fluidic channel in a region different from the region where the cells are focused in step a-iv) is carried out using a spectroscopic and/or microscopic method.

4. The method of any one of the preceding patent claims, wherein the lysing of at least the red blood cells is carried out using ultrasonic waves.

5. The method of any one of the preceding patent claims, wherein the lysing of at least the red blood cells is carried out in stopped flow.

6. The method of any one of the preceding patent claims, further comprising washing the fluidic channel after the analysis in step a-iv) with a suitable solvent and/or solution.

7. The method of any one of the preceding patent claims, wherein the fluidic channel is a microchannel with a height of between and including 5 and 500 µm, preferably between and including 10 and 200 µm, further preferably between and including 50 and 100 µm.

8. The method of any one of the preceding patent claims, wherein the second part of the blood sample introduced in step b1-ii) or b2-ii b) is diluted.

9. A device for analyzing a blood sample, comprising
- a fluidic channel (10);
- a first introducing element (11a, 11b) configured to introduce a first part of the blood sample into the fluidic channel;
- a focusing unit (12) configured to focus cells of the first part of the blood sample inside the fluidic channel into one predetermined region of the fluidic channel;
- at least a first analyzing unit (13a, 13b) configured to analyze the first part of the blood sample in a region different from the region where the cells are focused, including measuring at least one property w of the blood sample, particularly at least one of an extracellular hemoglobin content x, an extracellular lipid content y, and a maximum length z of clots; and
- an outputting interface (14) configured to automatically output at least a preliminary result of the analysis of the blood sample, wherein the preliminary result includes at least one property w of the blood sample, particularly at least one result of the extracellular hemoglobin content x, the extracellular lipid content y, and the maximum length z of clots, further comprising
- a decision module (15) configured to compare a measured result of the at least one property w of the blood sample to a reference value, particularly at least one of the extracellular hemoglobin content x, the extracellular lipid content y, and the maximum length z of clots, to a reference value of 150 mg/dL of extracellular hemoglobin content, a reference value of 600 mg/dL of extracellular lipid content, and a reference value of a maximum length of clots of 20 µm, respectively, wherein
if the result of the at least one property w of the blood sample is equal to or below the reference value, particularly if the result for extracellular hemoglobin content x is equal to or below the reference value of 150 mg/dL, and/or the result of extracellular lipid content y is equal to or below the reference value of 600 mg/dL, and/or the maximum length of clots z is equal to or less than 20 µm, the decision module is configured to provide an indication in the outputting interface that a second part of the blood sample can be measured, or is configured to provide no indication in the outputting interface, or
if the result of the at least one property w of the blood sample is above the reference value, particularly if the result for extracellular hemoglobin content x is above a reference value of 150 mg/dL, and/or the result of extracellular lipid content y is above a reference value of 600 mg/dL, and/or the maximum length of clots z is above 20 µm, the decision module is configured to provide an indication in the outputting interface that the blood sample is to be discarded and a new blood sample may need to be provided, or that the result may be erroneous;
further wherein the device comprises a second introducing element configured to introduce a second part of the blood sample into the fluidic channel, or wherein the first introducing element (11a, 11b) is further configured to also introduce a second part of the blood sample into the fluidic channel; and
further wherein the device comprises a lysing unit (16) configured to lyse at least red blood cells settled inside at least a part of the fluidic channel; or wherein the focusing unit (12) is further configured to lyse at least red blood cells settled inside at least a part of the fluidic channel.

10. The device of patent claim 9, wherein the focusing unit (12) is an acoustophoretic device.

11. The device of patent claim 10, wherein the acoustophoretic device can be configured to lyse at least red blood cells of the blood sample settled inside at least a part of the fluidic channel.

12. The device of any one of patent claims 9 to 11, wherein the at least first analyzing (13a, 13b) unit is also configured to analyze cells of the blood sample and/or products of a cell lysis of at least red blood cells of the blood sample, and/or further comprising at least a second analyzing unit configured to analyze the cells of the blood sample and/or products of a cell lysis of at least red blood cells of the blood sample.

13. The device of any one of patent claims 9 to 12, further comprising a flow control unit configured to control a flow inside of the fluidic channel (10).

14. The device of any one of patent claims 9 to 13, wherein the fluidic channel (10) is a microchannel (10') with a height of between and including 5 and 500 µm, preferably between and including 10 and 200 µm, further preferably between and including 50 and 100 µm.

15. A non-transient computer program product, configured to be used in a device of any one of patent claims 9 to 14 for analyzing a blood sample, wherein the non-transient computer program product is configured to carry out the following steps:
a-ii) introducing a first part of the blood sample into a fluidic channel;
a-iii) focusing cells of the first part of the blood sample inside the fluidic channel into one region of the fluidic channel;
a-iv) analyzing the first part of the blood sample in the fluidic channel in a region different from the region where the cells are focused, particularly a cell-free region, wherein analysis includes measuring at least one property w of the blood sample, particularly at least one of extracellular hemoglobin content x, extracellular lipid content y, and maximum length of clots z;
a-v) automatically outputting at least a preliminary result of the analysis of the first part of the blood sample, wherein the preliminary result includes the at least one property w of the blood sample, particularly at least one result of an extracellular hemoglobin content x, an extracellular lipid content y, and a maximum length of clots z,
wherein if the result of the at least one property w of the blood is equal to or below a reference value, particularly if the result for extracellular hemoglobin content x is equal to or below a value of 150 mg/dL, and/or the result of extracellular lipid content y is equal to or below a value of 600 mg/dL, and/or the maximum length of clots z is equal to or less than 20 µm, the following steps are carried out:
b1-i) flushing the first part of the blood sample from the fluidic channel;
b1-ii) introducing a second part of the blood sample into the fluidic channel;
b1-iii) settling cells of the second part of the blood sample inside at least a part of the fluidic channel;
b1-iv) optionally analyzing the settled cells at least for cell types;
b1-v) lysing at least red blood cells of the settled cells; and
b1-vi) analyzing at least the lysed red blood cells; or
wherein if the result of the at least one property w of the blood sample is above a reference value, particularly if the result for extracellular hemoglobin content x is above a value of 150 mg/dL, and/or the result of extracellular lipid content y is above a value of 600 mg/dL, and/or the maximum length of clots z is more than 20 µm, the following steps are carried out:
b2-i a) flushing the first part of the blood sample from the fluidic channel;
b2-ii a) automatically outputting a result that the blood sample is to be discarded and a new blood sample may need to be provided; or
b2-i b) flushing the first part of the blood sample from the fluidic channel;
b2-ii b) introducing a second part of the blood sample into the fluidic channel;
b2-iii b) settling of cells of the second part of the blood sample inside at least a part of the fluidic channel;
b2-iv b) optionally analyzing the settled cells at least for cell types;
b2-v b) lysing at least red blood cells of the settled cells;
b2-vi b) analyzing at least the lysed red blood cells, and
b2-vii b) outputting a result wherein it is indicated that the result may be erroneous.
